# EUROPÄISCHE PATENTSCHRIFT

(11) **EP 0 571 765 B1**
(45) Veröffentlichungstag und Bekanntmachung des Hinweises auf die Patenterteilung: **15.02.1995**
(21) Anmeldenummer: 93106703.7
(22) Anmeldetag: 26.04.1993
(51) Int. Cl.: G02C 5/22, A61F 9/02

(54) **Brille, insbesondere Arbeitsschutz- oder Sportbrille**
Glasses, in particular work safety goggles or sports glasses
Lunettes, spècialement protectrices pour travailleurs ou lunettes de sport

(30) Priorität: 26.05.1992 DE 9207109 U
(43) Veröffentlichungstag der Anmeldung: 01.12.1993
(73) Patentinhaber: UVEX WINTER OPTIK GmbH, D-90766 Fürth (DE)
(72) Erfinder: Wiedner, Klaus, W-8510 Fürth/Bay. (DE)
(74) Vertreter: Rau, Manfred, Dr. Dipl.-Ing.

(56) Entgegenhaltungen:
- EP-A- 0 076 499
- EP-A- 0 237 881
- DE-A- 1 925 231
- DE-B- 1 191 133
- DE-B- 1 281 711
- DE-U- 8 605 164
- DE-U- 8 902 696
- DE-U- 9 207 109

## Beschreibung

Die Erfindung richtet sich auf eine Brille, insbesondere eine Arbeitsschutz- oder Sportbrille, mit einer einteiligen, durchgehenden Scheibe und mit an einem Rahmenteil angelenkten, neigungsverstellbaren Bügeln.

Derartige Brillen weisen einerseits einen besonders einfachen, in reiner Kunststofftechnik realisierbaren Aufbau auf, andererseits ermöglicht eine so gestaltete Scheibe eine sehr gute Anpassung an die Anatomie des Trägers und einen zuverlässigen Schutz der Augen gegen Licht und auftreffende Partikel.

Zur Erhöhung des Tragekomforts und zur optimalen Anpassung an die Anatomie des Trägers ist es auch bekannt, die Bügel an der Scheibe neigungsverstellbar anzuordnen.

Bei einer an sich bewährten, vorbekannten Lösung ist beispielsweise vorgesehen, daß an seitlichen Ansätzen des Rahmens innenseitig Rastvorsprünge angeordnet sind, welche in Vertiefungen im Seitenbereich der Scheibe eingreifen.

Bei dieser Konstruktion besteht allerdings ein herstellungstechnisches Problem darin, daß zur Erzielung einer rationellen Fertigung die Rastvertiefungen mit dem Spritzen der Scheibe hergestellt werden müssen und dementsprechend beim anschließenden Aufbringen eines Überzuges auf die Scheibe, der zur Erhöhung der Kratzfestigkeit oder zur Erzielung von Antibeschlageigenschaften dienen kann, wieder überdeckt werden, so daß sie im anschließenden Gebrauchszustand, also nach dem Aushärten des Überzuges, nicht mehr tief genugt ausgeprägt sind, um eine zuverlässige, definierte Rastwirkung sicherzustellen.

Eine ähnliche Brille wird in DE-U-89 02 696.9 vorgestellt.

Hiervon ausgehend liegt der Erfindung die Aufgabe zugrunde, eine gattungsgemäße Brille so auszugestalten, daß bei geringem herstellungstechnischem Aufwand und unabhängig von dem Vorsehen einer Oberflächenbeschichtung der Scheibe in jedem Fall eine zuverlässige Einstellung und Beibehaltung der Bügelneigung relativ zur Scheibe gewährleistet ist.

Diese Aufgabe wird erfindungsgemäß durch die Merkmale von Anspruch 1 gelöst.

Durch diese Lösung dienen die halbkreisförmigen Schlitze einerseits als Führungsschlitze und gleichzeitig in Verbindung mit den Zapfen als Rastanordnung zur Einstellung eines bestimmten Inklinisationswinkels. Die so ausgebildeten Schlitze sind gegen die Aufbringung eines Coatings unempfindlich, d.h. es wird in jedem Fall und insbesondere auch nach längerer Benutzung eine funktionsfähige und zuverlässige Verstell- und Arretierwirkung erreicht.

Vorteilhafterweise ist vorgesehen, daß an der Innenseite der Zapfen in die Schlitze einführbare und diese im eingeführten Zustand übergreifende Halteansätze vorgesehen sind. Auf diese Weise wird sichergestellt, daß sich im normalen Gebrauchszustand das Rahmenteil nicht von der Scheibe löst.

Die horizontale Schwenkachse wird am einfachsten durch ein Schwenklager definiert, welches durch einen mittigen Zapfen an der Innenseite des Rahmenteils und eine Ausnehmung in der Mitte der Scheibe gebildet ist, wobei der Zapfen mit leichtem Spiel in diese Ausnehmung eingreift.

Zur Erzielung einer gewissen elastischen Nachgiebigkeit beim Verändern der Rastpositionen kann vorgesehen sein, daß parallel zu jedem die Rastlager ausbildenden Schlitz wenigstens ein weiterer Schlitz verläuft. Denkbar ist es insbesondere, beiderseits jedes die Rastlager bildenden Schlitzes einen parallel verlaufenden Schlitz vorzusehen. Hierdurch werden Stege ausgebildet, die den Rastlagerschlitz elastisch nachgiebig begrenzen. Insbesondere kann vorgesehen sein, daß die Kontur der Innenkante jedes äußeren Schlitzes der Kontur der Rastlagerschlitze folgt.

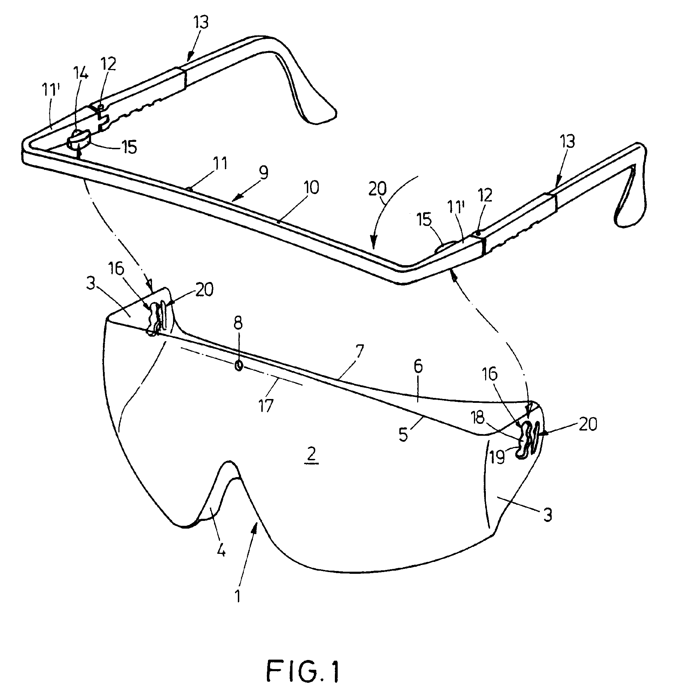

Nachfolgend wird die Erfindung anhand eines bevorzugten Ausführungsbeispieles in Verbindung mit der Zeichnung näher beschrieben. Dabei zeigen
- Fig. 1: eine perspektivische Ansicht einer erfindungsgemäßen Brille, wobei das Rahmenteil noch nicht an der Scheibe montiert ist und
- Fig. 2: eine Detailansicht des Schlitz-Bereiches.

Eine in der Zeichnung dargestellte Brille umfaßt eine aus Kunststoff einstückig gespritzte Scheibe 1, welche ein durchgehendes vorderes Sichtfeld 2 aufweist, an welches sich etwa in rechtem Winkel nach hinten abknickend seitliche Ansätze 3 anschließen. Im Bereich des Nasenrückens des Trägers ist ein die Auflagefläche vergrößerndes Nasenauflageteil 4 einstückig angeformt. Von der Oberkante 5 der Scheibe erstreckt sich ein im Tragezustand horizontaler Ansatz 6 mit einer der Stirnanatomie entsprechend gewölbten hinteren Kante 7 nach hinten.

Im Bereich der Mitte des Sichtfeldes 2 unterhalb der Oberkante 5 ist eine Lagerausnehmung 8 in Form einer kreisförmigen Bohrung ausgebildet.

Ein Rahmenteil 9 umfaßt einen Abschnitt 10, welcher im montierten Zustand parallel zur Oberkante 5 des Sichtfeldes 2 verläuft, wobei ein Lagerzapfen 11 in die Lagerausnehmung 8 eingreift. An den Rahmenabschnitt 10 schließen sich etwa um 90° nach hinten abknickend Rahmenansätze 11′ mit einem in herkömmlicher Weise ausgebildeten Scharnier 12 für im Ausführungsbeispiel längenverstellbare Bügel 13 an.

An der Innenseite der Rahmenansätze 11′ sind Zapfen 14 mit einem kopfartigen Halteansatz 15 ausgebildet.

Die seitlichen Rahmenansätze 11′ verlaufen parallel zu den seitlichen Ansätzen 3 der Scheibe 1 und umgreifen diese.

In den seitlichen Ansätzen 3 der Scheibe 1 sind Schlitze 16 ausgebildet, welche konzentrisch um die Schwenklagerachse 17 verlaufen, welche durch die Ausnehmung 8 und den Zapfen 11 definiert wird.

Die Schlitze 16 werden gebildet durch eine Mehrzahl sich schneidender, kreisförmiger Ausnehmungen 18, welche voneinander durch Rastvorsprünge 19 getrennt sind. Der Durchmesser der Ausnehmungen 18 entspricht etwa dem Durchmesser der Zapfen 14, so daß die Zapfen 14 unter Überwindung der Rastkraft aufgrund der Rastvorsprünge 19 von einer derartigen Ausnehmung 18 in die nächste verschwenkt werden können, wobei durch jede derartige Ausnehmung 18 ein bestimmter Neigungswinkel der Bügel, also ein definierter Inklinisationswinkel vorgegeben wird.

Zur Montage des Rahmenteils 9 an der Scheibe 1 wird das Rahmenteil gegenüber der in der Zeichnung dargestellten Gebrauchsposition um 90° verschwenkt, so daß dann die Halteansätze 15 die Schlitze 16 durchsetzen können. Schwenkt man anschließend das Rahmenteil 9 in Richtung des Pfeils 20, kann der Zapfen 11 in die Ausnehmung 8 einrasten und die Halteansätze 15 übergreifen die Schlitze 16, so daß ein stabiler Zustand erzielt wird, der gleichwohl ein Verschwenken der Bügel 13 einschließlich des Rahmenteils 10 relativ zur Scheibe 1 ermöglicht.

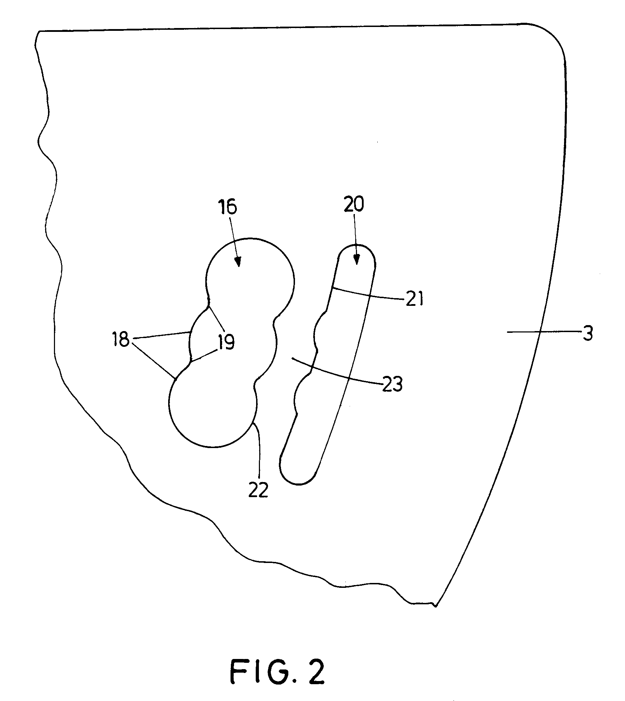

Wie insbesondere aus Fig. 2 erkennbar ist, erstreckt sich in einem gewissen Abstand parallel zu jedem Schlitz 16 ein weiterer Schlitz 20, dessen Innenkante 21 in ihrer Kontur etwa der Kontur der Kante 22 des Rast-Schlitzes 16 folgt, so daß zwischen den Schlitzen 16 und 20 ein Steg 23 ausgebildet wird, der beim Verstellen der Inklinationsposition, d.h. beim Übergang von einer Rastposition in die nächste, elastisch nachgeben kann. Durch die Dimensionierung des Steges 23 bzw. des Schlitzes 20, der als solcher auch noch auf der anderen Seite des Rast-Schlitzes 16 vorgesehen sein könnte, kann der Widerstand gegen die Verstellbarkeit oder mit anderen Worten die Rastkraft definiert eingestellt werden.

## Patentansprüche

1. Brille, insbesondere Arbeitsschutz- oder Sportbrille, mit einer einteiligen, durchgehenden Scheibe und mit an einem Rahmenteil angelenkten, neigungsverstellbaren Bügeln, dadurch gekennzeichnet,
daß die Scheibe (1) seitliche, nach hinten gezogene, etwa parallel zum Kopf des Trägers verlaufende, einstückig mit dieser ausgebildete Ansätze (3) aufweist,
daß das Rahmenteil (9) längs der Oberkante (5) der Scheibe (1) angeordnet ist und seitliche Ansätze (11′) aufweist, an denen die Bügel angelenkt sind, daß das Rahmenteil (9) und seine Ansätze (11′) die Scheibe (1) und deren Ansätze (3) umgreifen,
daß das Rahmenteil (9) mit seinen Ansätzen und mit den angelenkten Bügeln (13) an der Scheibe (1) um eine horizontale Schwenkachse (17) schwenkbar gelagert ist,
daß an jeder Innenseite der Rahmenansätze (11′) ein Zapfen (14) angeordnet ist, und
daß die Zapfen (14) in mit der Schwenkachse (17) konzentrische, halbkreisförmige Schlitze (16) in den Ansätzen (3) der Scheibe (1) eingreifen, wobei zur Festlegung der Zapfen (14) in bestimmten Winkelpositionen der Schlitze (16) diese durch eine Mehrzahl sich unter Ausbildung von Rastvorsprüngen (19) schneidenden, etwa kreisförmige Arretierausnehmungen (18) etwa entsprechend dem Querschnitt des jeweiligen Zapfens (14) gebildet sind.

2. Brille nach Anspruch 1, dadurch gekennzeichnet, daß am freien Ende jedes Zapfens (14) ein Halteansatz (15) angeordnet ist, der den Schlitz (16) übergreift.

3. Brille nach Anspruch 1, dadurch gekennzeichnet, daß in der Mitte der Scheibe (1) unterhalb von deren Oberkante (5) eine etwa kreisförmige Ausnehmung (8) angeordnet ist, in die ein Zapfen (11) an der Innenseite des Rahmenteils (9) unter Ausbildung eines Schwenklagers mit der Schwenkachse (17) eingreift.

4. Brille nach Anspruch 1, dadurch gekennzeichnet, daß parallel im Abstand zu dem Schlitz (16) mit den Rastvorsprüngen (19) wenigstens ein weiterer Schlitz (20) unter Ausbildung eines dazwischen liegenden, elastischen Steges (23) verläuft.

5. Brille nach Anspruch 4, dadurch gekennzeichnet, daß die Kontur der Innenkante (21) des weiteren Schlitzes (20) parallel zu der benachbarten Kante (22) des Schlitzes (16) verläuft.

## Claims

1. Glasses, in particular industrial safety or sports glasses, with an integral continuous sight piece and with inclination-adjustable side pieces articulated on a frame piece, characterized in
that the sight piece (1) has backwards oriented lateral appendixes (3) formed in one piece with it and extending about in parallel to the wearer's head,
in that the frame piece (9) is arranged along the upper edge (5) of the sight piece (1) and has lateral appendixes (11′) on which the side pieces are articulated, and that the frame piece (9) and its appendixes (11′) surround the sight piece (1) and the latter's appendixes (3),
in that the frame piece (9) with its appendixes and with the articulated side pieces (13) is supported on the sight piece (1) pivotably around a horizontal pivot axis (17),
in that a pin (14) is arranged on the inside of each of the appendixes (11′),
in that the pins (14) engage with semicircular slits (16) concentric of the pivot axis (17) in the appendixes (3) of the sight piece (1),
wherein, for the pins (14) to be arrested in certain angular positions of the slits (16), the latter are formed by a plurality of approximately annular locking recesses (18) intersecting to form locking protrusions (19) and approximately corresponding to the cross-section of the pin (14).

2. Glasses according to claim 1, characterized in that a retaining head (15), which overlaps the slit (16), is arranged at the free end of each pin (14).

3. Glasses according to claim 1, characterized in that in the middle of the sight piece (1) underneath its uppper edge (5), an approximately circular recess (8) is arranged, with which a pin (11) on the inside of the frame piece (9) engages, forming a pivot bearing with the pivot axis (17).

4. Glasses according to claim 1, characterized in that at least one further slit (20) extends at a distance in parallel to the slit (16) comprising the locking protrusions (19), forming an elastic web (23) located in between.

5. Glasses according to claim 4, characterized in that the contour of the inner edge (21) of the further slit (20) extends parallel to the adjacent edge (22) of the slit (16).

## Revendications

1. Lunettes, en particulier lunettes protectrices pour travailleurs ou lunettes de sport, comprenant un écran ininterrompu d'une seule pièce et des branches à inclinaison réglable articulées sur une partie formant cadre, caractérisées en ce que l'écran (1) comporte des pattes latérales (3) qui sont orientées vers l'arrière, s'étendent sensiblement parallèlement à la tête du porteur et sont réalisées d'une seule pièce avec ledit écran (1), en ce que la partie formant cadre (9) est disposée le long du bord supérieur (5) de l'écran (1) et comporte des pattes latérales (11′) sur lesquelles les branches sont articulées, en ce que la partie formant cadre (9) et ses pattes (11′) entourent l'écran (1) et ses pattes (3), en ce que la partie formant cadre (9) munie de ses pattes et des branches articulées (13) est montée pivotante sur l'écran (1) autour d'un axe de pivotement horizontal (17), en ce qu'un téton (14) est monté sur chaque côté intérieur des pattes de cadre (11′), et en ce que les tétons (14) s'engagent dans des fentes semi-circulaires (16) qui sont concentriques à l'axe de pivotement (17) et sont ménagées dans les pattes (3) de l'écran (1), les fentes (16) étant formées par une pluralité d'évidements d'arrêt sensiblement circulaires (18) qui se coupent en formant des crans (19) et qui correspondent sensiblement à la section transversale de chaque téton (14) afin d'immobiliser les tétons (14) dans des positions angulaires déterminées des fentes (16).

2. Lunettes selon la revendication 1, caractérisées en ce qu'à l'extrémité libre de chaque téton (14) se trouve une pièce de retenue (15) qui recouvre la fente (16).

3. Lunettes selon la revendication 1, caractérisées en ce qu'au milieu de l'écran (1), sous le bord supérieur (5) de celui-ci, est ménagé un évidement sensiblement circulaire (8) dans lequel un téton (11) disposé sur le côté intérieur de la partie formant cadre (9) s'engage en formant un pivot selon l'axe de pivotement (17).

4. Lunettes selon la revendication 1, caractérisées en ce qu'au moins une seconde fente (20) s'étend parallèlement et à distance de la fente (16) pourvue des crans (19), en formant entre elles une barrette élastique (23).

5. Lunettes selon la revendication 4, caractérisées en ce que le contour du bord intérieur (21) de la seconde fente (20) s'étend parallèlement au bord voisin (22) de la fente (16).
